# FASCICULE DE BREVET EUROPEEN

(11) **EP 1 301 176 B1**
(45) Date de publication et mention de la délivrance du brevet: **24.03.2004**
(21) Numéro de dépôt: 01956618.1
(22) Date de dépôt: 20.07.2001
(51) Int. Cl.: A61K 9/16, A61K 9/50

(54) **PROCEDE D'ENROBAGE DE PARTICULES SOLIDES PAR UN AGENT THERMOFUSIBLE, ET PARTICULES SOLIDES AINSI ENROBEES**
VERFAHREN ZUR UMHÜLLUNG VON FESTEN PARTIKELN MIT EINEM THERMOPLASTISCHEN HILFSTOFF, SOWIE DADURCH ÜBERZOGENE FESTE PARTIKEL
METHOD FOR COATING SOLID PARTICLES WITH A THERMOFUSIBLE AGENT, AND RESULTING COATED SOLID PARTICLES

(30) Priorité: 21.07.2000 FR 0009584
(43) Date de publication de la demande: 16.04.2003
(73) Titulaire: GATTEFOSSE HOLDING, 69800 St Priest (FR)
(72) Inventeur: BENAMEUR, Hassan, F-68140 Munster (FR); BARTHELEMY, Philippe, F-69780 Mions (FR)
(74) Mandataire: Vuillermoz, Bruno
(86) Numéro de dépôt international: PCT/FR2001/002365
(87) Numéro de publication internationale: WO 2002/007706

(56) Documents cités:
- US-A- 5 891 476
- A. FAHAM; ET AL.: "Hot melt coating technology: influence of Compritol 888 Ato and granule size on chloroquine release" DIE PHARMAZIE, vol. 55, juin 2000 (2000-06), pages 444-448, XP000932193

## Description

L'invention concerne un procédé d'enrobage de particules solides par un agent thermofusible. Elle se rapporte également aux particules solides enrobées ainsi obtenues.

Dans la suite de la description et dans les revendications, on désigne par l'expression « agent thermofusible », une matière première susceptible de passer sous l'effet de la chaleur d'un état solide à un état liquide en passant par un stade de ramollissement. Les températures de changement d'état varient bien entendu en fonction de la matière première utilisée.

De même, par l'expression "particules solides", on désigne des particules unitaires individualisées contenant un seul constituant à distinguer donc des granules contenant plusieurs constituants dont au moins un agent liant, destiné à lier les particules individualisées entre elles. Bien entendu, les particules de l'invention, lorsqu'elles sont utilisées en mélange, peuvent contenir chaune, un constituant de nature différente.

En tant que matière première de ce type, on décrit ci-après, l'utilisation de matière lipidique c'est à dire d'une matière à base d'acides gras libres et/ou d'esters d'acides gras.

La technique d'enrobage à chaud dite "hot melt coating" est une technique parfaitement connue de l'homme du métier, consistant, pour l'essentiel, à pulvériser, à chaud, sur des particules solides maintenues dans un lit d'air fluidisé, de fines gouttelettes d'une solution d'enrobage thermofusible.

Le document intitulé CHARACTERIZATION OF A HOT MELT FLUID BED COATING PROCESS FOR FINE GRANULES de JOZWIAKOWSKI, publié dans le journal Pharmaceutical Research Volume 7, Numéro 11 de 1990, met en oeuvre cette technique dans un appareillage commercialisé par GLATT sous la marque GPCG-5®. Dans ce type d'appareillage, la solution d'enrobage lipidique est pulvérisée à contre courant du flux d'air vertical ascendant maintenant les particules en suspension pour former le lit fluidisé. Plus précisément, la pulvérisation de l'agent d'enrobage est effectuée au sommet du lit d'air (top spray) à une pression d'atomisation d'air élevée, comprise entre 4 et 5 bars et à une température de la matière enrobante de 40 à 50° C plus élevée que son point de fusion (64° C). En outre, il est indiqué que la température du lit de poudre doit être maintenue voisine de la température de fusion de l'agent enrobant, soit dans le cas d'espèce, égale à 54° C.

On constate tout d'abord que ce procédé nécessite la mise en oeuvre de températures élevées. En outre, le fait que les gouttelettes de matière enrobante se déplacent à contre courant du flux des particules rend l'enrobage aléatoire et difficile à maîtriser. Cet enrobage aléatoire conduit à augmenter la proportion de matière enrobante pour espérer enrober les particules le plus régulièrement possible, augmentant ainsi le coût du procédé.

C'est ainsi que dans le document précité, l'enrobage est constitué de matière lipidique représentant 30 % en poids de la particule enrobée. En outre, si une telle proportion convient parfaitement pour contrôler la libération du principe actif, en revanche, il est incompatible avec une libération immédiate de celui-ci. Par ailleurs, la proportion importante d'agent d'enrobage réduit d'autant la concentration en principe actif de la formule pharmaceutique finale, conduisant à augmenter le poids de la forme finale, si une teneur élevée en principe actif est requise.

De plus, on observe qu'il est difficile de maintenir une température constante de la matière en fusion, tout d'abord en sortie de buse de pulvérisation, et ensuite dans le lit fluidisé, dans la mesure où ladite température diminue au moment de l'atomisation de la matière d'enrobage, puis au contact de l'air plus froid, arrivant à contre-courant. Une conséquence directe est qu'une partie de la matière lipidique se solidifie avant même d'entrer au contact des particules, réduisant la régularité de l'enrobage et conduisant à la formation d'une poudre fine solide d'agent enrobant. Ces phénomènes expliquent le choix d'une température supérieure de 50° C à la température de fusion de l'agent d'enrobage de sorte qu'il n'ait pas le temps de se solidifier avant son contact avec la particule à enrober et au contraire, parvienne dans un état parfaitement liquide supérieur à son point de fusion. Cependant, cette augmentation de température très au dessus de la température de fusion de l'agent enrobant peut conduire à un phénomène d'agglomération et donc de croissance de la taille des particules.

Un autre inconvénient de cette technique est de ne pouvoir obtenir un enrobage régulier de particules de faible diamètre, inférieur à 200 micromètres, sans conduire à des phénomènes d'agglomération des particules entre elles (granulation).

Enfin, on a constaté que ce procédé ne permettait pas d'enrober des particules themosensibles, en particulier celles dont la température de fusion est proche de celle de l'agent thermofusible, dans la mesure où l'agent thermofusible arrive au contact de la particule à une température largement supérieure à son point de fusion (environ 50° C plus élevé). En conséquence, on observe un ramollissement trop important de la particule pour permettre son enrobage. C'est ainsi qu'à la connaissance du demandeur, toutes les particules thermosensibles et en particulier tous les principes actif présentant un faible point de fusion sont enrobés, à froid, exclusivement par des polymères cellulosiques. Le document US-A-4 835 187 décrit par exemple un procédé d'enrobage de particules d'ibuprofène, dont le point de fusion est de 75° C par une solution d'éthylcellulose par technique dite de "spray drying".

Pour pallier les problèmes liés au mouvement des particules par rapport à celui de l'agent d'enrobage et l'enrobage aléatoire qui en résulte, on a proposé de pulvériser la matière thermofusible sur les particules, non pas à contre-courant du flux d'air, mais dans le même sens que ledit flux d'air, c'est-à-dire selon un mouvement vertical ascendant (principe du bottom spray, dont une technique de mise en oeuvre est dénommée WURSTER). Cependant, les résultats ne sont pas satisfaisants notamment lorsqu'il s'agit d'enrober de fines particules. En effet, le mouvement d'air mis en oeuvre provoque une accélération des particules en bloc conduisant à leur agglomération (granulation). Par ailleurs, cette technique ne permet pas de résoudre davantage le problème mis en évidence en top spray, qui est celui de la solidification de la matière lipidique au contact du courant d'air froid.

Le document FAHAM, publié dans DIE PHARMAZIE vol. 55, juin 2000, pages 444-448, décrit un troisième type de procédé d'enrobage consistant non pas à pulvériser la solution d'enrobage à contre courant ou dans le même sens que le flux d'air vertical ascendant mais perpendiculairement audit flux d'air, comme le montre la figure 1 de ce document. Cette technique est connue sous le nom anglais de "Tangential spray" et conduite dans un appareillage commercialisé par GLATT sous la dénomination GPCG1. Cet appareil est muni, comme le montre la figure, d'un disque tournant, le flux d'air circulant selon un mouvement ascendant entre le bord du disque et la paroi de l'appareil. La rotation du disque à grande vitesse confère au produit à enrober une force centrifuge, qui a pour effet de coller ou d'agglomérer le produit contre la paroi de l'appareil. Un tel procédé présente donc l'inconvénient d'augmenter la taille du produit à enrober par un phénomène de granulation avant l'enrobage proprement dit. En outre, l'enrobage tel que décrit est effectué sur des granules obtenus par granulation préalable, et non sur des particules individualisées solides au sens de l'invention. Il est donc impossible de procéder, par cette technique, à de l'enrobage de particules de faible diamètre, inférieur à 200 µm. On observe d'ailleurs que le pourcentage de particules enrobées à 6% dont la taille est inférieure à 200 µm, diminue de près de la moitié par rapport aux particules non enrobés (Table 2). Par ailleurs, ce procédé ne permet pas d'enrober des particules thermosensibles du fait, comme précédemment indiqué, que l'agent thermofusible arrive au contact du granule à une température largement supérieure à son point de fusion.

Dès lors, le premier problème que se propose de résoudre l'invention est de développer un procédé de "hot melt coating", qui puisse être conduit à des températures réduites et mieux contrôlées pour permettre de réduire l'énergie consommée.

Un second problème que se propose de résoudre l'invention est de développer un procédé qui permette d'obtenir un enrobage de matière thermofusible uniforme et régulier de particules solides en mettant en oeuvre une quantité de matière première la plus faible possible en fonction de l'objectif recherché.

Ainsi, par exemple, lorsque la particule solide est un principe actif, l'objectif de l'invention est d'enrober la particule avec le moins de matière possible, que ce soit pour obtenir une libération immédiate ou contrôlée du principe actif.

Un troisième problème que se propose de résoudre l'invention est de développer un procédé d'enrobage qui puisse s'appliquer à des particules de faible taille, en pratique inférieure à 200 micromètres, sans nécessiter de granulation préalable.

Un quatrième problème que se propose de résoudre l'invention est de développer un procédé d'enrobage qui puisse être appliqué à des particules thermosensibles, dont le point de fusion est proche, mais supérieur au point de fusion de l'agent d'enrobage.

Pour ce faire, l'invention propose un procédé d'enrobage de particules solides par au moins un agent thermofusible selon lequel :
- on fluidise les particules solides dans un mouvement d'air ascendant tournant en spirale permettant d'obtenir une répartition individualisée homogène des particules dans le lit d'air, la température du lit d'air étant inférieure à la température de fusion de l'agent thermofusible,
- on pulvérise ensuite sur les particules l'agent thermofusible fondu sous forme de gouttelettes atomisées, lesdites gouttelettes étant réparties dans un cône de pulvérisation contenu dans une zone d'air, dont la température permet de maintenir, tout au long de ladite pulvérisation, une température de l'agent thermofusible sensiblement égale à sa température de fusion, la pulvérisation étant effectuée de manière ascendante dans le même sens et tangentiellement au mouvement suivi par les particules solides ;
- enfin, lorsque l'enrobage est terminé, on refroidit les particules enrobées obtenues de manière à solidifier l'agent thermofusible autour des particules.

Ce procédé peut être mis en oeuvre au sein d'un appareil du type de celui décrit dans le document US-A-5 282 321 reproduisant tant le mouvement des particules que celui de l'agent d'enrobage décrit précédemment

En d'autres termes, l'invention consiste à combiner une première étape de fluidisation des particules solides selon un mouvement permettant d'obtenir une individualisation et une répartition des particules parfaitement homogènes à une seconde étape de pulvérisation tangentielle, également ascendante et dans le même sens, dans des conditions telles que l'agent thermofusible proche de sa température de fusion puisse être en contact immédiat avec les particules, réduisant ainsi tout risque de refroidissement rapide et donc de solidification prématurée de l'agent thermofusible. Ce procédé permet non seulement d'obtenir un enrobage uniforme, mais également de travailler à des températures proches de la température de fusion de l'agent thermofusible.

En outre, le maintien de la température de l'agent thermofusible proche de son point de fusion tout au long de la pulvérisation permet d'enrober des particules thermosensibles, dont le point de fusion est proche, mais supérieur au point de fusion de l'agent enrobant. En effet, l'agent enrobant arrive au contact de la particule à l'état ramolli, correspondant à son point de fusion, et non à l'état liquide, correspondant à une température supérieure, de sorte qu'il n'est pas suffisamment chaud pour modifier l'état physique de la particule.

En outre, le mouvement spécifique des particules au sein du lit d'air, lesquelles restent individualisées sans phénomène d'agglomération associée à une pulvérisation effectué selon un mouvement similaire, permet d'enrober des particules individualisées de faible diamètre, inférieur à 200 micromètres, avantageusement entre 30 et 180 micromètres. Bien entendu, la täille des particulès inférieure à 200 micromètres n'est pas un facteur limitant à la réalisation du procédé, celui-ci pouvant être mis en oeuvre pour des tailles de particules supérieures. Par ailleurs, il convient de mentionner que le diamètre indiqué correspond au diamètre moyen d'une population de particules.

Pour diminuer l'importance du ramollissement de la particule au contact de l'agent thermofusible en fusion, la température du lit d'air est avantageusement choisie de sorte à maintenir la particule solide et son environnement à une température en dessous de la température de fusion de l'agent thermofusible, avantageusement d'une valeur voisine de 20° C inférieure à la température de fusion de l'agent thermofusible. Bien entendu, la température de l'air peut varier de quelques degrés tout au long du procédé notamment lorsque l'agent thermofusible entre en contact avec les particules solides.

Pour maintenir à sa température de fusion l'agent thermofusible tout au long de l'étape de pulvérisation, la température de la zone d'air entourant le cône de pulvérisation dans lequel sont maintenues les gouttelettes atomisées est avantageusement choisie entre + ou - 5° C par rapport à la température de fusion de l'agent thermofusible.

Selon une autre caractéristique du procédé de l'invention, la pression d'air d'atomisation de l'agent thermofusible est préalablement fixée entre 0,3 bar et 5 bars, avantageusement entre 1 et 2 bars. Bien entendu, l'homme du métier adaptera la pression d'atomisation en fonction de la nature et des caractéristiques rhéologiques de l'enrobage à pulvériser.

Par ailleurs, la température de l'air d'atomisation de l'agent thermofusible est au maximum de 10° C supérieure à la température de fusion dudit agent.

Selon une autre caractéristique, la pression de la zone d'air entourant le cône contenant les gouttelettes atomisées est de préférence inférieure à 1,5 bar, avantageusement égale à 0,5 bar.

Par ailleurs et selon une autre caractéristique, le débit de pulvérisation de l'agent thermofusible est compris entre 5 et 500 g/minute. Là encore, l'homme du métier réglera le débit en fonction de la nature et des caractéristiques rhéologiques de l'agent d'enrobage, ainsi qu'en fonction de la masse des particules à enrober et de leur taille.

Ainsi, par exemple, pour une masse à enrober de 2 000 g, dont la taille des particules constitutives est comprise entre 30 et 180 micromètres, le débit de pulvérisation sera avantageusement choisi entre 5 et 50 g/minute.

Un autre avantage de l'invention est de réduire la proportion de l'agent d'enrobage et donc le coût de la composition dans la mesure où la répartition homogène des particules dans le lit fluidisé combiné à la maîtrise de la température de l'agent d'enrobage conduit à obtenir un enrobage régulier.

En pratique, l'enrobage représente de 1 à 25 % en poids de la particule enrobée en fonction de l'objectif recherché. Ainsi, l'enrobage représente entre 5 et 8 %, lorsqu'il a pour objectif de masquer le goût d'un principe actif, et 10 à 20 %, lorsqu'il a pour objectif de prolonger la libération d'un principe actif.

Bien entendu, le procédé de l'invention concerne n'importe quel type de particules solides destinées à être enrobées.

Cependant et dans une forme de réalisation avantageuse, la particule solide est un principe actif choisi dans le groupe comprenant : hydrochlorothiazide, acetazolamide, acide acétylsalicylique, allopurinol, alprenolol, amiloride, antiarhythmique, antibiotique, antidiabétique, antiépilectique, anticoagulants, antimycotique, atenolol, bendroflumethiazide, benzbromarone, benzthiazide, bétaméthasone, et leurs esters, bronchodilateur, buphenine, bupranolol, chlordiazepoxide, chloroquine, chlorothiazide, chlorpromazine, chlortalidone, clenbuterol, clomipramine, clonidine, co-dergocrine, cortisone, et leurs esters, dexaméthasone, et leurs esters, dextropropoxyphène, diazepam, diazoxide, diclofenac, diclofenamide, digitalisglycoside, dihydralazine, dihidroergotamine, diltiazem, , sels métalliques, ergotamine, acide ethacrynique, éthinylestradiol, éthoxzolamide, fénotérol, fludrocortinone, et leurs esters, fluphenazine, furorosemide, gallopamil, guanethidine, hormone, hydrocortisone, et leurs esters, hydroflumethiazide, immunosuppresseur, ibuprofène, imipranine, indométhacine, levodopa, sel de lithium, sel de magnésium, acétate de médroxyprogestérone, ménadione, méthaqualone, 8-méthoxypsoralen, méthylclothiazide, méthyldopa, méthylprednisolone, méthylestostérone, méthylthiouracil, méthylxanthine, métipranodol, molsidomine, morphine, naproxène, nicergoline, nifédipine, norfénéfrine, oxyphenbutazone, papavérine, parmathasone, et leurs esters, pentobarbital, perphenazine, phénobarbital, phénylbutazone, phytoménadione, pirenzepine, polythiazide, prazosine, prednisolone, et leurs esters, prednisone, et leurs esters, probenecid, propranolol, propylthiouracil, rescinnamine, réserpine, secbutabarbital, sécobarbital, spironolactone, sulfasalazine, sulfonamide, thioridazine, triamcinolone, et leurs esters, triamteren, trichlormethiazide, trifluoperazine, trifluopromazine, tuberculostastique, vérapamil, virustatique, zytostatique, bromocriptine, bromopride, carbidopa, carbocromen, quinine, chlorprothixène, cimétidine, clofibrate, cyclizine, désipramine, disulfiram, dompéridone, doxepine, fenbufen, acide flufénamine, flumarizine, gemfibrocil, halöpéridol, kétoprofène, labétalol, lorazepam, acide méfénamine, melpérone, métoclopramide, nortriptyline, noscapine, oxprenolol, oxymétholone, pentazocine, péthidine, stanozolol, sulindac, sulpiride, tiotixène.

Par ailleurs et comme déjà dit, on désigne par l'expression « agent thermofusible », une matière première susceptible de passer de l'état solide à l'état liquide en passant par un ramollissement sous l'effet de la température.

Dans une forme de réalisation avantageuse du procédé, l'agent thermofusible est un lipide, c'est-à-dire une matière première à base d'acides gras libres et/ou d'esters d'acides gras, de préférence comprenant au moins un ester partiel d'alcool avec au moins un acide gras.

Selon une première forme de réalisation, le lipide est un ester d'acide béhénique et d'alcool commercialisé par le demandeur sous la marque COMPRITOL ®. La température de fusion du COMPRITOL ® varie entre 69 et 74° C et permet d'enrober des particules thermosensibles de point de fusion proche, mais supérieur, par exemple l'ibuprofène dont le point de fusion est égal à 75° C.

Dans une seconde forme de réalisation, l'agent lipidique est un ester d'acide palmitostéarique et d'alcool, commercialisé sous la marque PRECIROL ATO 5 ® et dont le point de fusion varie entre 53 et 57° C.

Bien entendu, l'invention concerne la particule solide enrobée susceptible d'être obtenue par le procédé décrit ci-avant.

L'invention a également pour objet une particule solide enrobée par un agent d'enrobage comprenant au moins un ester partiel d'alcool avec au moins un acide gras. Cette particule se caractérise en ce que sa taille avant enrobage est inférieure 400 µm, avantageusement inférieure à 200 micromètres et en ce que l'enrobage représente entre 1 et 25% en poids de la particule enrobée.

Dans une forme de réalisation avantageuse, l'enrobage représente de 2 à 8 % en poids de la particule enrobée.

Selon une autre caractéristique, la particule est thermosensible et présente un point de fusion proche, mais supérieur à celui de l'agent thermofusible.

Selon un mode de réalisation particulier, la particule est un principe actif choisi dans le groupe des principes actifs précédemment cités.

L'enrobage est de nature lipidique et choisi préférentiellement dans le groupe comprenant les esters d'acide palmitostéarique et d'alcool et les esters d'acide béhénique et d'alcool.

L'invention concerne également toute composition intégrant les particules enrobées ci-avant décrites.

Dans un mode de réalisation particulier, l'invention a pour objet une particule d'ibuprofène enrobée par un agent d'enrobage qui se caractérise en ce que la taille de la particule non enrobée est inférieure à 200 micromètres et en ce que l'agent d'enrobage comprend au moins un ester partiel d'alcool avec au moins un acide gras et représente entre 1 et 25 % en poids de la particule enrobée, avantageusement entre 2 et 8 %.

Bien entendu et de même que précédemment le diamètre des particules défini ci-avant correspond à un diamètre moyen d'une population de particules donnée.

Dans une forme de réalisation avantageuse, l'agent d'enrobage est choisi dans le groupe comprenant les esters d'acide palmitostéarique et d'alcool et les esters d'acide béhénique et d'alcool.

Bien entendu, les particules enrobées peuvent être intégrées directement dans des sachets, gélules ou incorporées dans des comprimés, sans que cette liste soit limitative.

L'invention et les avantages qui en découlent ressortiront mieux des exemples de réalisation ci-après à l'appui des figures annexées selon lesquelles :
- la figure 1 est une représentation de la distribution d'un lots de particules d'ibuprofène enrobées et non enrobées ;
- la figure 2 est une représentation de la distribution de plusieurs lots de particules d'ibuprofène enrobées et non enrobées ;

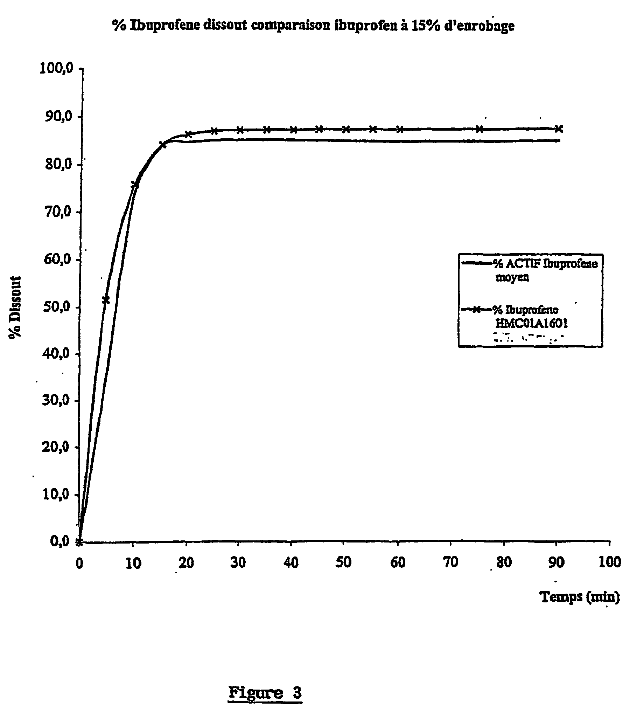

- la figure 3 est une courbe de dissolution d'ibuprofène enrobé et non enrobé;
- la figure 4 est une représentation de la distribution des particules sphériques de résines échangeuses d'ions (REI) non enrobées (poudre A) et enrobées (poudre B) au moyen d'un histogramme de distribution ( 4a) et d'une courbe de distribution cumulée ( 4b) ;
- la figure 5 est une représentatnion de la distribution de lots de particules de paracétamol enrobés et non enrobés.

### EXEMPLE 1 : IBUPROFENE ENROBE

Dans cet exemple, on enrobe 2 000 g d'ibuprofène dont le diamètre moyen des particules est égal à 176 micromètres par 146 g de PRECIROL ATO 5®. L'enrobage représentant donc 7 % en poids du poids total de la particule enrobée. On rappelle que la température de fusion du PRECIROL ATO 5® est comprise entre 52 et 57° C alors que la température de fusion de l'ibuprofène est égale à 75° C.

Le procédé est mis en oeuvre dans un appareil dénommé KUGELCOATER ® commercialisé par la société HUTTLIN. Le modèle de KUGELCOATER ® utilisé est le UNILAB-05.

Dans cet exemple, les caractéristiques mises en oeuvre tout au long du procédé sont représentées dans le tableau ci-après.

| Durée (mn) | Débit d'air (Nm³/h) | Temp. lit d'air (°C) | Temp. Particules (°C) | débit de pulvérisation (g/mn) | Pression atomisation ± 0,1 (bar) | Pression zone d'air entourant le cône de pulvérisation (bar) | Temp. Air d'atomisation (°C) | Temp. zone d'ai entourant cône de pulvérisa tion (°C) |
|---|---|---|---|---|---|---|---|---|
| 1 | 158 | 35,0 | 36,0 | 6 | 1,0 | 0.5 | 60 | 50 |
| 4 | 161 | 34,6 | 38,4 | 6 | 1,0 | 0.5 | 60 | 50 |
| 10 | 171 | 35,0 | 39,7 | 6 | 1,0 | 0.5 | 60 | 50 |
| 15 | 168 | 35,3 | 40,1 | 6 | 1,0 | 0.5 | 60 | 50 |
| 23 | 166 | 35,3 | 40,1 | 6 | 1,0 | 0.4 | 60 | 50 |
| 32 | 142 | 22,0 | 33,2 | 6 | 0,4 | 0.4 | 60 | 50 |
| 41 | 145 | 19,7 | 30,5 | 6 | 0,4 | 0.4 | 60 | 50 |

Comme le montre le tableau, on diminue la pression d'atomisation à partir de la 32^{e} minute pour permettre le refroidissement.

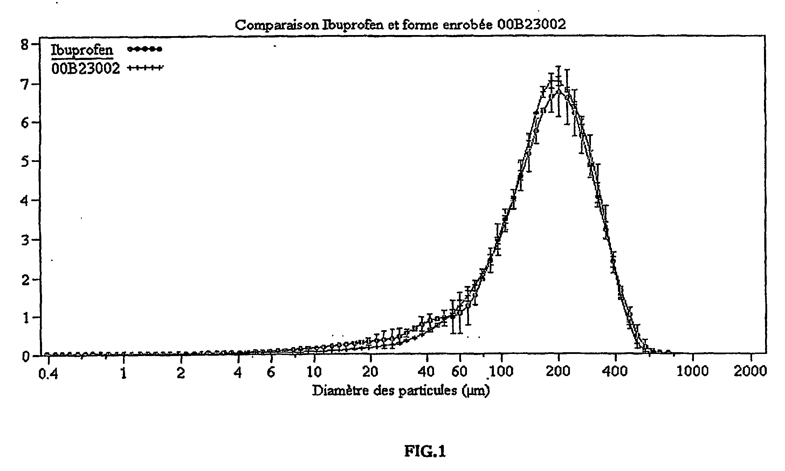

Sur la figure 1, on a représenté la répartition des particules avant et après enrobage. Comme le montre cette figure, l'ensemble des particules enrobées présente la même distribution que celle des particules non enrobées, montrant non seulement que les particules ont été régulièrement enrobées mais également que la quantité d'enrobage est moindre. Ainsi, on observe que le diamètre moyen des particules avant enrobage est égal à 176 micromètres, alors que le diamètre moyen des particules enrobées est égal à 180 micromètres.

On constate ainsi que le procédé permet d'enrober à chaud des particules dont le point de fusion est proche de celui de l'agent d'enrobage.

### EXEMPLE 2 :

Le but de cette caractérisation est d'évaluer la qualité de l'enrobage obtenu sur six lots d'Ibuprofène enrobés.

Les lots étudiés sont référencés par la suite HMC01A1601 / HMC01A1602 /HMC01A1603 / HMC01A1604 / HMC07A1605 / HMC01A1606, fabriqués à partir du principe actif Ibuprofène EP lot 520011014 enrobé de PRECIROL® ARO 5 lot 23907 à raison de 15 %. Le procédé est mis en oeuvre dans un appareil identique à celui utilisé dans l'exemple 1 avec les mêmes caractéristiques (débit, pression etc ...)

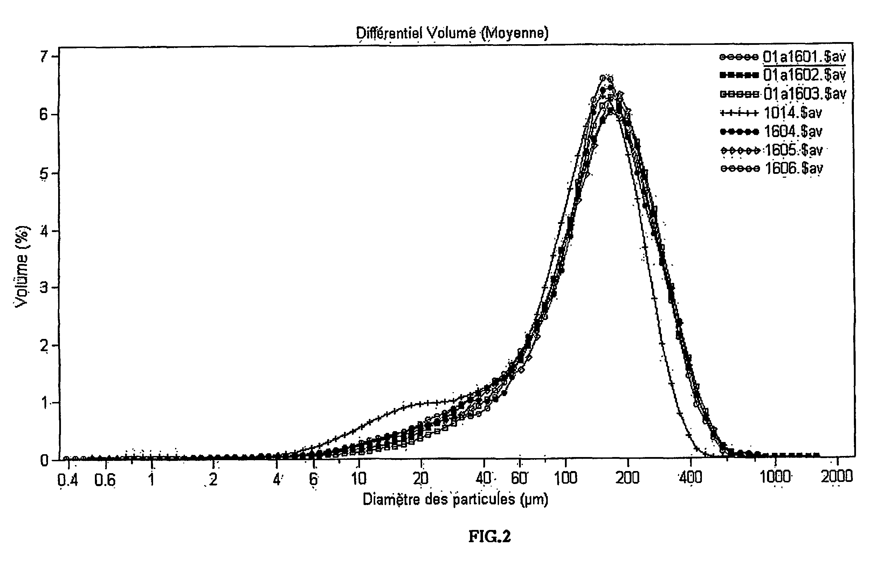

Dans le tableau suivant, on a représenté la taille moyenne des particules d'ibuprofène (D50) avant et après enrobage. La figure 2 représente parallèlement la répartition des particules avant et après enrobage.

| Echantillon | Moyenne (µm) |
|---|---|
| 5200I1014 | 130.0 |
| HMC01A1601 | 161.0 |
| HMC01A1602 | 165.2 |
| HMC01A1603 | 169.7 |
| HMC01A1604 | 164.7 |
| HMC01A1605 | 164.7 |
| HMC01A1606 | 160.6 |

Comme le montre cette figure, l'ensemble des particules enrobées présente la même distribution que celles des particules non enrobées, prouvant non seulement que les particules ont été régulièrement enrobées mais également que la qualité d'enrobage est moindre. En effet, le diamètre des particules avant enrobage est de 130 µm alors qu'après enrobage, il est d'au plus 169.7 µm (lot HMC01A1603).

Un test de dissolution d'ibuprofène enrobé et non enrobé (lot HMC01A1601) a également été effectué conformément aux instructions de la pharmacopée européenne.

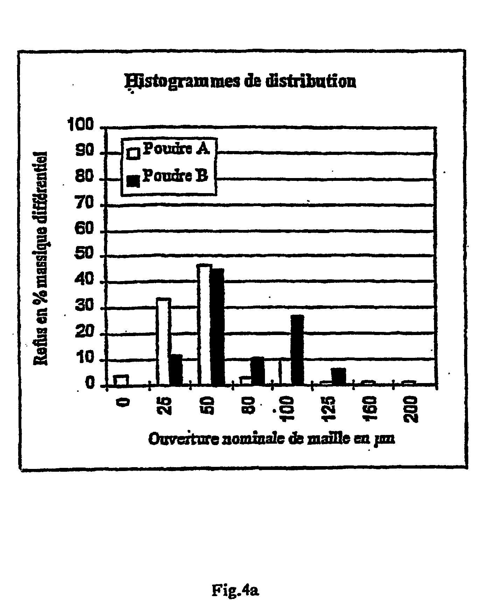

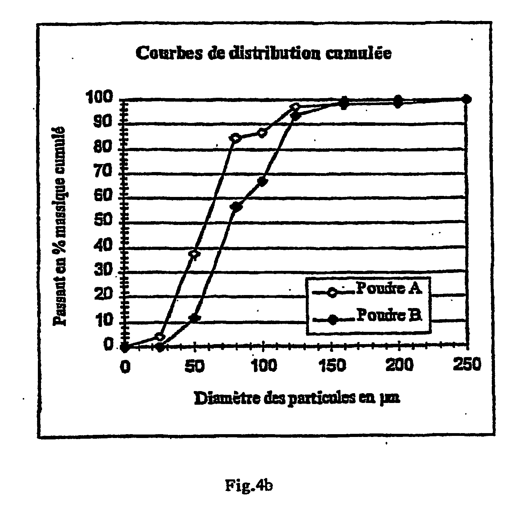

Les résultats apparaissent sur la figure 4. Comme le montre cette figure, la vitesse de dissolution de l'ibuprofène enrobé est pratiquement identique à celle de l'ibuprofène seul, ce qui prouve que l'enrobage a peut d'influence sur la libération du principe actif.

### EXEMPLE 3 : RESINE ECHANGEUSE D'IONS (REI) ENROBEE

Dans cet exemple, on enrobe 2 000 g de REI, dont le diamètre moyen des particules est égal à 60 micromètres par 350 g de COMPRITOL®. L'enrobage représente donc 17,5% en poids du poids total de la particule enrobée.

Le procédé est mis en oeuvre dans un appareil identique à celui précédemment utilisé.

Dans cet exemple, les caractéristiques mises en oeuvre tout au long du procédé sont représentées dans le tableau ci-après.

| Durée (mn) | débit d'air (Nm³/h) | Temp. lit d'air (°C) | Temp. Particules (°C) | débit de pulvérisation (g/mn) | Pression atomisation ± 0,1 (bar) | Pression zone d'air entourant le cône de pulvérisation (bar) | Temp. Air d'atomisation (°C) | Temp. zone d'air entourant cône de pulvérisation (°C) |
|---|---|---|---|---|---|---|---|---|
| 3 | 157 | 64,8 | 56,5 | 23 | 1,6 | 1,03 | 72 | 60 |
| 20 | 190 | 45,0 | 56,8 | 11 | 1,6 | 1,05 | 72 | 60 |
| 35 | 178 | 45,1 | 55,8 | 11 | 1,58 | 1,03 | 72 | 60 |
| 48 | 167 | 43,0 | 50,9 | 11 | 1,58 | 1,03 | 72 | 60 |

Sur la figure 2, on a représenté la répartition des particules avant et après enrobage.

Comme le montre cette figure, l'ensemble des particules enrobées présente la même distribution que celles des particules non enrobées, montrant non seulement que les particules ont été régulièrement enrobées, mais également que la quantité d'enrobage est moindre. Ainsi, on observe que le diamètre moyen des particules avant enrobage est égal à 60 micromètres, alors que le diamètre moyen des particules enrobées est égal à 75 micromètres.

### EXEMPLE 4 :

Le but de cette caractérisation est d'évaluer la qualité de l'enrobage obtenu sur quatre lots pilotes de paracetamol enrobés.

Les lots étudiés sont HMC01A1707/ HMC01A1708/ HMC01A1709/ HMC01A1710, fabriqués à partir du principe actif Paracetamol Rhodia lot 99292402 et l'enrobant est du Precirol ATO 5 lot 23907 en quantité théorique de 6% massique.

Le procédé est mis en oeuvre dans un appareil identique à celui de l'exemple 1 et dans les mêmes conditions.

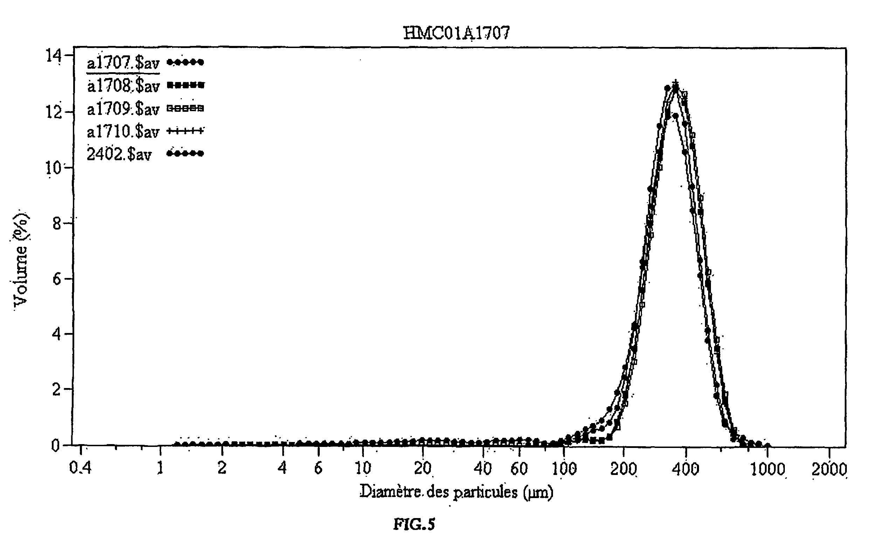

Dans le tableau suivant, on a représenté la taille moyenne des particules de paracetamol avant et après enrobage. La figure 5 représente parallèlement la répartition des particules avant et après enrobage.

| Echantillon | Médiane (µm) |
|---|---|
| 99292402 | 326.1 |
| HMC01A1707 | 336.1 |
| HMC01A1708 | 355.2 |
| HMC01A1709 | 361.2 |
| HMC01A1710 | 354.8 |

Comme le montre cette figure, l'ensemble des particules enrobées présente la même distribution que celles des particules non enrobées, prouvant non seulement que les particules ont été régulièrement enrobées mais également que la qualité d'enrobage est moindre. En effet, le diamètre des particules avant enrobage est de 326.1 µm alors qu'après enrobage, il est d'au plus 361.2 µm (lot HMC01A1709).

L'invention et les avantages qui en découlent ressortent bien de la description. On notera en particulier la possibilité d'enrober des particules thermosensibles, dont la température de fusion est proche de celle de l'agent enrobant, ce qui n'était pas possible avec les techniques existantes. La technique décrite permet en outre de diminuer l'énergie nécessaire au procédé. Par ailleurs, le procédé permet d'enrober de façon régulière des particules de diamètre faible, inférieur à 200 micromètres, ce qui n'était pas possible avec les quantités décrites d'agent enrobant par d'autres techniques.

## Revendications

1. Procédé d'enrobage de particules solides par au moins un agent thermofusible selon lequel :
• on fluidise les particules solides dans un mouvement d'air ascendant tournant en spirale permettant d'obtenir une répartition individualisée homogène des particules dans le lit d'air, la température du lit d'air étant inférieure à la température de fusion de l'agent thermofusible,
• on pulvérise ensuite sur les particules l'agent thermofusible fondu sous forme de gouttelettes atomisées, lesdites gouttelettes étant réparties dans un cône de pulvérisation contenu dans une zone d'air, dont la température permet de maintenir, tout au long de ladite pulvérisation, une température de l'agent thermofusible sensiblement égale à sa température de fusion, la pulvérisation étant effectuée de manière ascendante dans le même sens et tangentiellement au mouvement suivi par les particules solides ;
• enfin, lorsque l'enrobage est terminé, on refroidit les particules enrobées obtenues de manière à solidifier l'agent thermofusible autour des particules.

2. Procédé selon la revendication 1, **caractérisé en ce que** la particule solide est thermosensible et présente un point de fusion proche, mais supérieure à celui de l'agent thermofusible.

3. Procédé selon la revendication 1, **caractérisé en ce que** le diamètre des particules solides est inférieur à 200 micromètres, avantageusement compris entre 30 et 180 micromètres.

4. Procédé selon la revendication 1, **caractérisé en ce que** la température du lit d'air est choisie de sorte à maintenir la particule solide à une température en dessous de la température de fusion de l'agent thermofusible, avantageusement d'une valeur voisine de 20° C inférieure à la température de fusion de l'agent thermofusible.

5. Procédé selon la revendication 1, **caractérisé en ce que** la pression d'air d'atomisation de l'agent thermofusible est préalablement fixée entre 0,3 bar et 5 bars, avantageusement entre 1 et 2 bars.

6. Procédé selon la revendication 1, **caractérisé en ce que** la température de la zone d'air entourant le cône de pulvérisation dans lequel sont maintenues les gouttelettes atomisées est avantageusement choisie entre + ou - 5° C par rapport à la température de fusion de l'agent thermofusible.

7. Procédé selon la revendication 1, **caractérisé en ce que** la pression de la zone d'air entourant le cône de pulvérisation contenant les gouttelettes atomisées est inférieure à 1,5 bar, avantageusement égale à 0,5 bar.

8. Procédé selon la revendication 1, **caractérisé en ce que** la température de l'air d'atomisation de l'agent thermofusible est au maximum de 10° C supérieure à la température de fusion dudit agent.

9. Procédé selon la revendication 1, **caractérisé en ce que** le débit de pulvérisation de l'agent thermofusible est compris entre 5 et 50 g/minute.

10. Procédé selon la revendication 1, **caractérisé en ce que** l'enrobage représente de 1 à 25 % en poids, en fonction de l'objectif recherché.

11. Procédé selon la revendication 1, **caractérisé en ce que** la particule solide est un principe actif choisi dans le groupe comprenant : hydrochlorothiazide, acetazolamide, acide acétylsalicylique, allopurinol, alprenolol, amiloride, antiarhythmique, antibiotique, antidiabétique, antiépilectique, anticoagulants, . antimycotique, atenolol, bendroflumethiazide, benzbromarone, benzthiazide, bétaméthasone, et leurs esters, bronchodilateur, buphenine, bupranolol, chlordiazepoxide, chloroquine, chlorothiazide, chlorpromazine, chlortalidone, clenbuterol, clomipramine, clonidine, co-dergocrine, cortisone, et leurs esters, dexaméthasone, et leurs esters, dextropropoxyphène, diazepam, diazoxide, diclofenac, diclofenamide, digitalisglycoside, dihydralazine, dihidroergotamine, diltiazem, , sels métalliques, ergotamine, acide ethacrynique, éthinylestradiol, éthoxzolamide, fénotérol, fludrocortinone, et leurs esters, fluphenazine, furorosemide, gallopamil, guanethidine, hormone, hydrocortisone, et leurs esters, hydroflumethiazide, immunosuppresseur, ibuprofène, imipranine, indométhacine, levodopa, sel de lithium, sel de magnésium, acétate de médroxyprogestérone, ménadione, méthaqualone, 8-méthoxypsoralen, méthylclothiazide, méthyldopa, méthylprednisolone, méthylestostérone, méthylthiouracil, méthylxanthine, métipranodol, molsidomine, morphine, naproxène, nicergoline, nifédipine, norfénéfrine, oxyphenbutazone, papavérine, parmathasone, et leurs esters, pentobarbital, perphenazine, phénobarbital, phénylbutazone, phytoménadione, pirenzepine, polythiazide, prazosine, prednisolone, et leurs esters, prednisone, et leurs esters, probenecid, propranolol, propylthiouracil, rescinnamine, réserpine, secbutabarbital, sécobarbital, spironolactone, sulfasalazine, sulfonamide, thioridazine, triamcinolone, et leurs esters, triamteren, trichlormethiazide, trifluoperazine, trifluopromazine, tuberculostastique, vérapamil, virustatique, zytostatique, bromocriptine, bromopride, carbidopa, carbocromen, quinine, chlorprothixène, cimétidine, clofibrate, cyclizine, désipramine, disulfiram, dompéridone, doxepine, fenbufen, acide flufénamine, flunarizine, gemfibrocil, halopéridol, kétoprofène, labétalol, lorazepam, acide méfénamine, melpérone, métoclopramide, nortriptyline, noscapine, oxprenolol, oxymétholone, pentazocine, péthidine, stanozolol, sulindac, sulpiride, tiotixène.

12. Procédé selon la revendication 1, **caractérisé en ce que** l'agent thermofusible est un lipide à base d'acides gras libres et/ou d'esters d'acides gras.

13. Procédé selon la revendication 12, **caractérisé en ce que** le lipide comprend au moins un ester partiel d'alcool avec au moins un acide gras.

14. Procédé selon la revendication 13, **caractérisé en ce que** le lipide est choisi dans le groupe comprenant les esters d'acide palmitostéarique et d'alcool et les esters d'acide béhénique et d'alcool.

15. Particule solide enrobée susceptible d'être obtenue par le procédé objet de la revendication 1.

16. Particule solide enrobée par un agent d'enrobage comprenant au moins un ester partiel d'alcool avec au moins un acide gras, **caractérisée** ce que la taille de la particule avant enrobage est inférieure à 400micromètres, avantageusement inférieure à 200 µm et en ce que l'enrobage représente entre 1 et 25 % en poids de la particule enrobée.

17. Particule selon la revendication 16, **caractérisée en ce que** l'enrobage représente de 2 à 8 % en poids de la particule enrobée.

18. Particule selon la revendication 16, **caractérisée en ce qu'**elle est thermosensible et présente un point de fusion proche, mais supérieur à celui de l'agent thermofusible.

19. Particule selon la revendication 16, **caractérisée en ce que** la particule est un principe actif choisi dans le groupe comprenant : hydrochlorothiazide, acetazolamide, acide acetylsalicylique, allopurinol, alprenolol, amiloride, antiarhythmique, antibiotique, antidiabétique, antiépilectique, anticoagulants, antimycotique, atenolol, bendroflumethiazide, benzbromarone, benzthiazide, bétaméthasone, et leurs esters, bronchodilateur, buphenine, bupranolol, chlordiazepoxide, chloroquine, chlorothiazide, chlorpromazine, chlortalidone, clenbuterol, clomipramine, clonidine, co-dergocrine, cortisone, et leurs esters, dexaméthasone, et leurs esters, dextropropoxyphène, diazepam, diazoxide, diclofenac, diclofenamide, digitalisglycoside, dihydralazine, dihidroergotamine, diltiazem, , sels métalliques, ergotamine, acide ethacrynique, éthinylestradiol, éthoxzolamide, fénotérol, fludrocortinone, et leurs esters, fluphenazine, furorosemide, gallopamil, guanethidine, hormone, hydrocortisone, et leurs esters, hydroflumethiazide, immunosuppresseur, ibuprofene, imipranine, indométhacine, levodopa, sel de lithium, sel de magnésium, acétate de médroxyprogestérone, ménadione, méthaqualone, 8-méthoxypsoralen, méthylclothiazide, méthyldopa, méthylprednisolone, méthylestostérone, méthylthiouracil, méthylxanthine, métipranodol, molsidomine, morphine, naproxène, nicergoline, nifédipine, norfénéfrine, oxyphenbutazone, papavérine, parmathasone, et leurs esters, pentobarbital, perphenazine, phénobarbital, phénylbutazone, phytoménadione, pirenzepine, polythiazide, prazosine, prednisolone, et leurs esters, prednisone, et leurs esters, probenecid, propranolol, propylthiouracil, rescinnamine, réserpine, secbutabarbital, sécobarbital, spironolactone, sulfasalazine, sulfonamide, thioridazine, triamcinolone, et leurs esters, triamteren, trichlormethiazide, trifluoperazine, trifluopromazine, tuberculostastique, vérapamil, virustatique, zytostatique, bromocriptine, bromopride, carbidopa, carbocromen, quinine, chlorprothixène, cimétidine, clofibrate, cyclizine, désipramine, disulfiram, dompéridone, doxepine, fenbufen, acide flufénamine, flunarizine, gemfibrocil, halopéridol, kétoprofene, labétalol, lorazepam, acide méfénamine, melpérone, métoclopramide, nortriptyline, noscapine, oxprenolol, oxymétholone, pentazocine, péthidine, stanozolol, sulindac, sulpiride, tiotixène.

20. Particule selon la revendication 16, **caractérisée** ce que l'ester partiel d'alcool avec au moins un acide gras est choisi dans le groupe comprenant les esters d'acide palmitostéarique et d'alcool et les esters d'acide béhénique et d'alcool.

21. Composition intégrant les particules enrobées objets de la revendication 16.

22. Particule d'ibuprofène enrobée par un agent d'enrobage selon la revendication 15 ou 16, **caractérisée en ce que** la taille de la particule non enrobée est inférieure à 200 micromètres et **en ce que** l'agent d'enrobage comprend au moins un ester partiel d'alcool avec au moins un acide gras et représente entre 1 et 25 % en poids de la particule enrobée, avantageusement entre 2 et 8%.

23. Particule selon la revendication 22, **caractérisé en ce que** l'agent d'enrobage est choisi dans le groupe comprenant les esters d'acide palmitostéarique et d'alcool et les esters d'acide béhénique et d'alcool.

## Patentansprüche

1. Beschichtungsverfahren für feste Teilchen durch mindestens einen Heißschmelzstoff, wobei:
• die festen Teilchen in einer aufsteigenden spiralförmig drehenden und Luftbewegung fluidisiert werden, um eine homogene vereinzelte Verteilung der Teilchen in dem Wirbelbett zu erzielen, wobei die Temperatur des Wirbelbetts unter der Schmelztemperatur des Heißschmelzstoffs liegt;
• anschließend auf die Teilchen der geschmolzene Heißschmelzstoff in der Form zerstäubter Tröpfchen aufgesprüht wird, wobei diese Tröpfchen in einem Zerstäubungskegel verteilt sind, der sich in einer Luftzone befindet, deren Temperatur es ermöglicht, während der gesamten Zerstäubungsdauer eine Temperatur des Heißschmelzstoffs beizubehalten, die im Wesentlichen gleich seiner Schmelztemperatur ist, wobei die Zerstäubung aufsteigend in derselben Richtung und tangential zu der von den festen Teilchen verfolgten Bewegung erfolgt;
• wenn schließlich die Beschichtung abgeschlossen ist, die erhaltenen beschichteten Teilchen abgekühlt werden, um den Heißschmelzstoff, der die Teilchen umgibt, zu verfestigen.

2. Verfahren nach Anspruch 1, **dadurch gekennzeichnet, dass** das feste Teilchen wärmeempfindlich ist und einen Schmelzpunkt aufweist, der nahe aber über dem des Heißschmelzstoffs liegt.

3. Verfahren nach Anspruch 1, **dadurch gekennzeichnet, dass** der Durchmesser der festen Teilchen unter 200 Mikrometer beträgt, vorteilhafterweise zwischen 30 und 180 Mikrometer.

4. Verfahren nach Anspruch 1, **dadurch gekennzeichnet, dass** die Temperatur des Wirbelbetts so gewählt ist, dass das feste Teilchen bei einer Temperatur unterhalb der Schmelztemperatur des Heißschmelzstoffs, vorteilhafterweise bei einem Wert von ungefähr 20 °C unter der Schmelztemperatur des Heißschmelzstoffs, gehalten wird.

5. Verfahren nach Anspruch 1, **dadurch gekennzeichnet, dass** der Druck der Zerstäubungsluft des Heißschmelzstoffs zuvor zwischen 0,3 und 5 bar eingestellt wird, vorteilhafterweise zwischen 1 und 2 bar.

6. Verfahren nach Anspruch 1, **dadurch gekennzeichnet, dass** die Temperatur der Luftzone, die den Zerstäubungskegel umgibt, in dem sich die zerstäubten Tröpfchen befinden, vorteilhafterweise zwischen + oder -5 °C in Bezug auf die Schmelztemperatur des Heißschmelzstoffs gewählt ist.

7. Verfahren nach Anspruch 1, **dadurch gekennzeichnet, dass** der Druck der Luftzone, die den die zerstäubten Tröpfchen enthaltenden Zerstäubungskegel umgibt, weniger als 1,5 bar und vorteilhafterweise 0,5 bar beträgt.

8. Verfahren nach Anspruch 1, **dadurch gekennzeichnet, dass** die Temperatur der Zerstäubungsluft des Heißschmelzstoffs maximal 10 °C über der Schmelztemperatur des besagten Stoffs liegt.

9. Verfahren nach Anspruch 1, **dadurch gekennzeichnet, dass** die Zerstäubungsleistung des Heißschmelzstoffs zwischen 5 und 50 g/Minute beträgt.

10. Verfahren nach Anspruch 1, **dadurch gekennzeichnet, dass** die Beschichtung je nach angestrebtem Ziel 1 bis 25 Gew.-% ausmacht.

11. Verfahren nach Anspruch 1, **dadurch gekennzeichnet, dass** das feste Teilchen ein Wirkstoff ist, ausgewählt aus der Gruppe, bestehend aus: Hydrochlorthiazid, Acetazolamid, Acetylsalicylsäure, Allopurinol, Alprenolol, Amilorid, Antiarhythmikum, Antibiotikum, Antidiabetikum, Antiepilektikum, Antikoagulant, Antimycotikum, Atenolol, Bendroflumethiazid, Benzobromaron, Benzthiazid, Betamethason, und deren Ester, Bronchodilator, Buphenin, Bupranolol, Chlordiazepoxid, Chlorochin, Chlorothiazid, Chlorpromazin, Chlortalidon, Clenbuterol, Clomipramin, Clonidin, Codergocrin, Kortison, und deren Ester, Dexamethason, und deren Ester, Dextropropoxyphen, Diazepam, Diazoxid, Diclofenac, Diclofenamid, Digitalisglycosid, Dihydralazin, Dihidroergotamin, Diltiazem, anorganische Salze, Ergotamin, Ethacrynsäure, Ethinylestradiol, Ethoxzolamid, Fenoterol, Fludrocortison, und deren Ester, Fluphenazin, Fluorosemid, Gallopamil, Guanethidin, Hormon, Hydrocortison, und deren Ester, Hydroflumethiazid, Immunsuppressor, Ibuprofen, Imipranin, Indomethacin, Levodopa, Lithiumsalz, Magnesiumsalz, Medroxyprogesteronacetat, Menadion, Methaqualon, 8-Methoxypsoralen, Methylclothiazid, Methyldopa, Methylprednisolon, Methylestosteron, Methylthiouracil, Methylxanthin, Metipranodol, Molsidomin, Morphin, Naproxen, Nicergolin, Nifedipin, Norfenefrin, Oxyphenbutazon, Papaverin, Parmathason, und deren Ester, Pentobarbital, Perphenazin, Phenobarbital, Phenylbutazon, Phytomenadion, Pirenzepin, Polythiazid, Prazosin, Prednisolon, und deren Ester, Prednison, und deren Ester, Probenecid, Propranolol, Propylthiouracil, Rescinnamin, Reserpin, Secbutabarbital, Secobarbital, Spironolacton, Sulfasalazin, Sulfonamid, Thioridazin, Triamcinolon, und deren Ester, Triamteren, Trichlormethiazid, Trifluoperazin, Trifluopromazin, Tuberculostaticum, Verapamil, Virustaticum, Zytostaticum, Bromocriptin, Bromoprid, Carbidopa, Carbocromen, Chinin, Chlorprothixen, Cimetidin, Clofibrat, Cyclizin, Desipramin, Disulfiram, Domperidon, Doxepin, Fenbufen, Flufenaminsäure, Flunarizin, Gemfibrocil, Haloperidol, Ketoprofen, Labetalol, Iorazepam, Mefenaminsäure, Melperon, Metoclopramid, Nortriptylin, Noscapin, Oxprenolol, Oxymetholon, Pentazocin, Pethidin, Stanozolol, Sulindac, Sulpirid, Tiotixen.

12. Verfahren nach Anspruch 1, **dadurch gekennzeichnet, dass** der Heißschmelzstoff ein Fettstoff auf der Basis freier Fettsäuren und/oder Fettsäureestern ist.

13. Verfahren nach Anspruch 12, **dadurch gekennzeichnet, dass** der Fettstoff mindestens einen Teilalkoholester mit mindestens eine Fettsäure enthält.

14. Verfahren nach Anspruch 13, **dadurch gekennzeichnet, dass** der Fettstoff ausgewählt ist aus der Gruppe, die die Palmstearinsäure- und Alkoholester und Behensäure- und Alkoholester enthält.

15. Festes beschichtetes Teilchen, geeignet um mittels des Verfahrens nach Anspruch 1 hergestellt zu werden.

16. Festes Teilchen, beschichtet mit einem Beschichtungsstoff, der mindestens einen Teilalkoholester mit mindestens eine Fettsäure enthält, **dadurch gekennzeichnet, dass** die Größe des Teilchens vor dem Beschichten unter 400 Mikrometer liegt, vorteilhafterweise unter 200 µm, und dass die Beschichtung zwischen 1 und 25 Gew.-% des beschichteten Teilchens ausmacht.

17. Teilchen nach Anspruch 16, **dadurch gekennzeichnet, dass** die Beschichtung zwischen 2 und 8 Gew.-% des beschichteten Teilchens ausmacht.

18. Teilchen nach Anspruch 16, **dadurch gekennzeichnet, dass** es wärmeempfindlich ist und einen Schmelzpunkt aufweist, der nahe aber über dem des Heißschmelzstoffs liegt.

19. Teilchen nach Anspruch 16, **dadurch gekennzeichnet, dass** dieses Teilchen ein Wirkstoff ist, ausgewählt aus der Gruppe, bestehend aus: Hydrochlorthiazid, Acetazolamid, Acetylsalicylsäure, Allopurinol, Alprenolol, Amilorid, Antiarhythmikum, Antibiotikum, Antidiabetikum, Antiepilektikum, Antikoagulant, Antimycotikum, Atenolol, Bendroflumethiazid, Benzobromaron, Benzthiazid, Betamethason, und deren Ester, Bronchodilator, Buphenin, Bupranolol, Chlordiazepoxid, Chlorochin, Chlorothiazid, Chlorpromazin, Chlortalidon, Clenbuterol, Clomipramin, Clonidin, Codergocrin, Kortison, und deren Ester, Dexamethason, und deren Ester, Dextropropoxyphen, Diazepam, Diazoxid, Diclofenac, Diclofenamid, Digitalisglycosid, Dihydralazin, Dihidroergotamin, Diltiazem, anorganische Salze, Ergotamin, Ethacrynsäure, Ethinylestradiol, Ethoxzolamid, Fenoterol, Fludrocortison, und deren Ester, Fluphenazin, Fluorosemid, Gallopamil, Guanethidin, Hormon, Hydrocortison, und deren Ester, Hydroflumethiazid, Immunsuppressor, Ibuprofen, Imipranin, Indomethacin, Levodopa, Lithiumsalz, Magnesiumsalz, Medroxyprogesteronacetat, Menadion, Methaqualon, 8-Methoxypsoralen, Methylclothiazid, Methyldopa, Methylprednisolon, Methylestosteron, Methylthiouracil, Methylxanthin, Metipranodol, Molsidomin, Morphin, Naproxen, Nicergolin, Nifedipin, Norfenefrin, Oxyphenbutazon, Papaverin, Parmathason, und deren Ester, Pentobarbital, Perphenazin, Phenobarbital, Phenylbutazon, Phytomenadion, Pirenzepin, Polythiazid, Prazosin, Prednisolon, und deren Ester, Prednison, und deren Ester, Probenecid, Propranolol, Propylthiouracil, Rescinnamin, Reserpin, Secbutabarbital, Secobarbital, Spironolacton, Sulfasalazin, Sulfonamid, Thioridazin, Triamcinolon, und deren Ester, Triamteren, Trichlormethiazid, Trifluoperazin, Trifluopromazin, Tuberculostaticum, Verapamil, Virustaticum, Zytostaticum, Bromocriptin, Bromoprid, Carbidopa, Carbocromen, Chinin, Chlorprothixen, Cimetidin, Clofibrat, Cyclizin, Desipramin, Disulfiram, Domperidon, Doxepin, Fenbufen, Flufenaminsäure, Flunarizin, Gemfibrocil, Haloperidol, Ketoprofen, Labetalol, Iorazepam, Mefenaminsäure, Melperon, Metoclopramid, Nortriptylin, Noscapin, Oxprenolol, Oxymetholon, Pentazocin, Pethidin, Stanozolol, Sulindac, Sulpirid, Tiotixen.

20. Teilchen nach Anspruch 16, **dadurch gekennzeichnet, dass** der Teilalkoholester mit mindestens einer Fettsäure ausgewählt ist aus der Gruppe, die die Palmstearinsäure- und Alkoholester und Behensäure- und Alkoholester enthält.

21. Verbindung, die die beschichteten Teilchen integriert, die Gegenstand von Anspruch 16 sind.

22. Ibuprofenteilchen, beschichtet mit einem Beschichtungsstoff gemäß Anspruch 15 oder 16, **dadurch gekennzeichnet, dass** die Größe der unbeschichteten Teilchen unter 200 Mikrometer liegt und dass der Beschichtungsstoff mindestens einen Teilalkoholester mit mindestens eine Fettsäure enthält und zwischen 1 und 25 Gew.-% des beschichteten Teilchens ausmacht, vorteilhafterweise zwischen 2 und 8 %.

23. Teilchen nach Anspruch 22, **dadurch gekennzeichnet, dass** der Beschichtungsstoff ausgewählt ist aus der Gruppe, die die Palmstearinsäure- und Alkoholester und Behensäure- und Alkoholester enthält.

## Claims

1. The process for coating solid particles with at least one hot-melt agent, according to which:
• the solid particles are fluidized in a spiralling, ascending current of air making it possible to obtain a homogeneous separated distribution of the particles in the air bed, the temperature of the air bed being lower than the melting temperature of the hot-melt agent,
• the molten hot-melt agent is then sprayed onto the particles, in the form of atomized droplets, said droplets being distributed in a spray cone contained in a region of air, the temperature of which makes it possible to maintain, throughout said spraying, a hot-melt agent temperature which is substantially equal to the melting temperature thereof, the spraying being carried out in an ascending manner in the same direction as and tangentially to the path of the solid particles,
• finally, when the coating is finished, the coated particles obtained are cooled so as to solidify the hot-melt agent around the particles.

2. The process as claimed in claim 1, **characterized in that** the solid particle is heat-sensitive and has a melting point close to, but higher than, that of the hot-melt agent.

3. The process as claimed in claim 1, **characterized in that** the diameter of the solid particles is less than 200 micrometers, advantageously between 30 and 180 micrometers.

4. The process as claimed in claim 1, **characterized in that** the temperature of the air bed is chosen so as to maintain the solid particle at a temperature which is below the melting temperature of the hot-melt agent, and which advantageously has a value close to 20°C lower than the melting temperature of the hot-melt agent.

5. The process as claimed in claim 1, **characterized in that** the air pressure for atomizing the hot-melt agent is set, beforehand, between 0.3 bar and 5 bar, advantageously between 1 and 2 bar.

6. The process as claimed in claim 1, **characterized in that** the temperature of the region of air surrounding the spray cone in which the atomized droplets are maintained is advantageously chosen between + or -5°C with respect to the melting temperature of the hot-melt agent.

7. The process as claimed in claim 1, **characterized in that** the pressure of the region of air surrounding the spray cone containing the atomized droplets is less than 1.5 bar, advantageously equal to 0.5 bar.

8. The process as claimed in claim 1, **characterized in that** the temperature of the air for atomizing the hot-melt agent is a maximum of 10°C higher than the melting temperature of said agent.

9. The process as claimed in claim 1, **characterized in that** the rate of spraying the hot-melt agent is between 5 and 50 g/minute.

10. The process as claimed in claim 1, **characterized in that** the coating represents from 1 to 25% by weight, depending on the objective sought.

11. The process as claimed in claim 1, **characterized in that** the solid particle is an active principle chosen from the group comprising: hydrochlorothiazide, acetazolamide, acetylsalicylic acid, allopurinol, alprenolol, amiloride, an anti-arrhythmia agent, an antibiotic, an antidiabetic, an anti-epileptic, anti-clotting agents, an antimycotic agent, atenolol, bendroflumethiazide, benzbromarone, benzthiazide, betamethasone and the esters thereof, a bronchodilator, buphenine, bupranolol, chlordiazepoxide, chloroquine, chlorothiazide, chlorpromazine, chlortalidone, clenbuterol, clomipramine, clonidine, co-dergocrine, cortisone, and the esters thereof, dexamethasone, and the esters thereof, dextropropoxyphene, diazepam, diazoxide, diclofenac, diclofenamide, digitalis glycoside, dihydralazine, dihidroergotamine, diltiazem, metal salts, ergotamine, ethacrynic acid, ethinyloestradiol, ethoxyzolamide, fenoterol, fludrocortinone, and the esters thereof, fluphenazine, furosemide, gallopamil, guanethidine, a hormone, hydrocortisone, and the esters thereof, hydroflumethiazide, an immunosuppressor, ibuprofen, imipramine, indomethacin, levodopa, a lithium salt, a magnesium salt, medroxyprogesterone acetate, menadione, methaqualone, 8-methoxypsoralen, methylclothiazide, methyldopa, methylprednisolone, methylestosterone, methylthiouracil, methylxanthine, metipranodol, molsidomine, morphine, naproxen, nicergoline, nifedipine, norfenefrine, oxyphenbutazone, papaverine, parmathasone, and the esters thereof, pentobarbital, perphenazine, phenobarbital, phenylbutazone, phytomenadione, pirenzepine, polythiazide, prazosine, prednisolone, and the esters thereof, prednisone, and the esters thereof, probenecid, propranolol, propylthiouracil, rescinnamine, reserpine, secbutabarbital, secobarbital, spironolactone, sulphasalazine, sulphonamide, thioridazine, triamcinolone, and the esters thereof, triamteren, trichlormethiazide, trifluoperazine, trifluopromazine, a tubercular static agent, verapamil, a virustatic agent, a zytostatic agent, bromocriptine, bromopride, carbidopa, carbocromen, quinine, chlorprothixene, cimetidine, clofibrate, cyclizine, desipramine, disulphiram, domperidone, doxepin, fenbufen, flufenamine acid, flunarizine, gemfibrocil, haloperidol, ketoprofen, labetalol, lorazepam, mefenamine acid, melperone, metoclopramide, nortriptyline, noscapine, oxprenolol, oxymetholone, pentazocine; pethidine, stanozolol, sulindac, sulpiride, tiotixene.

12. The process as claimed in claim 1, **characterized in that** the hot-melt agent is a lipid based on free fatty acids and/or on fatty acid esters.

13. The process as claimed in claim 12, **characterized in that** the lipid comprises at least one partial ester of alcohol with at least one fatty acid.

14. The process as claimed in claim 13, **characterized in that** the lipid is chosen from the group comprising esters of palmitostearic acid and of alcohol, and esters of behenic acid and of alcohol.

15. A coated solid particle which can be obtained using the process which is the subject of claim 1.

16. A solid particle coated with a coating agent comprising at least one partial ester of alcohol with at least one fatty acid, **characterized in that** the particle size before coating is less than 400 micrometers, advantageously less than 200 micrometers, and **in that** the coating represents between 1 and 25% by weight of the coated particle.

17. The particle as claimed in claim 16, **characterized in that** the coating represents from 2 to 8% by weight of the coated particle.

18. The particle as claimed in claim 16, **characterized in that** it is heat-sensitive and has a melting point which is close to, but higher than, that of the hot-melt agent.

19. The particle as claimed in claim 16, **characterized in that** the particle is an active principle chosen from the group comprising: hydrochlorothiazide, acetazolamide, acetylsalicylic acid, allopurinol, alprenolol, amiloride, an anti-arrhythmia agent, an antibiotic, an antidiabetic, an anti-epileptic, anti-clotting agents, an antimycotic agent, atenolol, bendroflumethiazide, benzbromarone, benzthiazide, betamethasone and the esters thereof, a bronchodilator, buphenine, bupranolol, chlordiazepoxide, chloroquine, chlorothiazide, chlorpromazine, chlortalidone, clenbuterol, clomipramine, clonidine, co-dergocrine, cortisone, and the esters thereof, dexamethasone, and the esters thereof, dextropropoxyphene, diazepam, diazoxide, diclofenac, diclofenamide, digitalis glycoside, dihydralazine, dihidroergotamine, diltiazem, metal salts, ergotamine, ethacrynic acid, ethinyloestradiol, ethoxyzolamide, fenoterol, fludrocortinone, and the esters thereof, fluphenazine, furosemide, gallopamil, guanethidine, a hormone, hydrocortisone, and the esters thereof, hydroflumethiazide, an immunosuppressor, ibuprofen, imipramine, indomethacin, levodopa, a lithium salt, a magnesium salt, medroxyprogesterone acetate, menadione, methaqualone, 8-methoxypsoralen, methylclothiazide, methyldopa, methylprednisolone, methylestosterone, methylthiouracil, methylxanthine, metipranodol, molsidomine, morphine, naproxen, nicergoline, nifedipine, norfenefrine, oxyphenbutazone, papaverine, parmathasone, and the esters thereof, pentobarbital, perphenazine, phenobarbital, phenylbutazone, phytomenadione, pirenzepine, polythiazide, prazosine, prednisolone, and the esters thereof, prednisone, and the esters thereof, probenecid, propranolol, propylthiouracil, rescinnamine, reserpine, secbutabarbital, secobarbital, spironolactone, sulphasalazine, sulphonamide, thioridazine, triamcinolone, and the esters thereof, triamteren, trichlormethiazide, trifluoperazine, trifluopromazine, a tubercular static agent, verapamil, a virustatic agent, a zytostatic agent, bromocriptine, bromopride, carbidopa, carbocromen, quinine, chlorprothixene, cimetidine, clofibrate, cyclizine, desipramine, disulphiram, domperidone, doxepin, fenbufen, flufenamine acid, flunarizine, gemfibrocil, haloperidol, ketoprofen, labetalol, lorazepam, mefenamine acid, melperone, metoclopramide, nortriptyline, noscapine, oxprenolol, oxymetholone, pentazocine, pethidine, stanozolol, sulindac, sulpiride, tiotixene.

20. The particle as claimed in claim 16, **characterized in that** the partial ester of alcohol with at least one fatty acid is chosen from the group comprising esters of palmitostearic acid and of alcohol, and esters of behenic acid and of alcohol.

21. A composition which integrates the coated particles which are the subjects of claim 16.

22. An ibuprofen particle coated with a coating agent as claimed in claims 15 or 16, **characterized in that** the uncoated particle size is less than 200 micrometers, and **in that** the coating agent comprises at least one partial ester of alcohol with at least one fatty acid and represents between 1 and 25% by weight of the coated particle, advantageously between 2 and 8%.

23. The particle as claimed in claim 22, **characterized in that** the coating agent is chosen from the group comprising esters of palmitostearic acid and of alcohol, and esters of behenic acid and of alcohol.
